(19) Europäisches Patentamt / European Patent Office / Office européen des brevets

(11) **EP 4 681 755 A1**

(12) **DEMANDE DE BREVET EUROPEEN**

(43) Date de publication:
21.01.2026 Bulletin 2026/04

(51) Classification Internationale des Brevets (IPC):
*A61M 16/00* (2006.01)   *A61M 16/20* (2006.01)

(21) Numéro de dépôt: 25182596.4

(22) Date de dépôt: **13.06.2025**

(52) Classification Coopérative des Brevets (CPC):
**A61M 16/204; A61M 16/024; A61M 16/125; A61M 16/205;** A61M 16/0066; A61M 2016/0027; A61M 2016/0033; A61M 2202/0208; A61M 2205/3331; A61M 2205/3372; F16K 31/0675; G05D 7/0635

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **19.07.2024 FR 2407952**

(71) Demandeur: **Air Liquide Medical Systems 92182 Antony Cedex (FR)**

(72) Inventeur: **BECHIKHI, Taraq 92182 Antony (FR)**

(74) Mandataire: **Air Liquide L'Air Liquide S.A. Direction de la Propriété Intellectuelle 75, Quai d'Orsay 75321 Paris Cedex 07 (FR)**

Remarques:
Revendications modifiées conformément à la règle 137(2) CBE.

(54) **APPAREIL DE VENTILATION À ÉLECTROVANNE PROPORTIONNELLE À OUVERTURE CONTRÔLÉE**

(57)     L'invention concerne un appareil de ventilation (1) comprenant un circuit de gaz interne (2) pour véhiculer un gaz, et au moins une électrovanne proportionnelle (4, 40, 400) avec un orifice de passage de gaz (4.1) à degré d'ouverture ajustable, agencée sur le circuit de gaz interne et comprenant une bobine (4.2) alimentée électriquement. Des moyens de pilotage électroniques (5) contrôlent le degré d'ouverture de l'orifice de passage de gaz de l'électrovanne proportionnelle en agissant sur le courant électrique fourni à la bobine de l'électrovanne proportionnelle de manière à délivrer un débit ou une pression de gaz désiré. Les moyens de pilotage électroniques comprennent un circuit électronique (10) configuré pour ajuster l'intensité du courant électrique fourni à la bobine en fonction d'une consigne de tension préfixée (Vset).

Fig. 1

Fig. 2

EP 4 681 755 A1

# EP 4 681 755 A1

**Description**

L'invention concerne un appareil de ventilation comprenant une ou plusieurs électrovannes proportionnelles dont l'ouverture est contrôlée grâce à un circuit électronique particulier permettant d'ajuster l'intensité du courant électrique fourni à la bobine de la ou des électrovannes proportionnelles en fonction d'une consigne de tension.

Dans les appareils médicaux de ventilation, aussi appelés respirateurs artificiels ou ventilateurs médicaux, on utilise une ou des électrovannes proportionnelles pour contrôler, i.e. réguler ou ajuster, les pressions et/ou les débits de gaz au sein de l'appareil.

Pour ce faire, on fait varier proportionnellement le degré d'ouverture (i.e. diamètre, taille ou dimensions) de l'orifice de passage du gaz (i.e. orifice calibré) de l'électrovanne proportionnelle en fonction de la pression ou du débit souhaité.

Pour pouvoir modifier le degré d'ouverture de l'orifice, par exemple son diamètre/taille, on fait varier le courant électrique dans la bobine de l'électrovanne en commandant la bobine en tension soit en tension variable, soit en tension fixe avec hachage du courant.

Autrement dit, la commande d'une (ou de) électrovanne proportionnelle est habituellement basée sur un contrôle, par un circuit électronique, de l'alimentation électrique de sa bobine avec une tension variable ou constante et avec hachage du courant de la bobine avec un signal rectangulaire modulé en largeur d'impulsion de manière à faire varier le courant envoyé à la bobine et à contrôler l'ouverture de l'orifice de l'électrovanne.

Des exemples de ventilateur contrôlés selon l'art antérieur sont donnés par WO00/32261, WO2024/210552 et WO2024/210566.

L'inconvénient de ces manières de commander la bobine d'une électrovanne proportionnelle est la non-compensation de la dérive systématique du courant (i.e. intensité) réglé lorsque la bobine s'échauffe par effet joule et/ou en raison d'une augmentation de la température ambiante.

Or, toute élévation de température de la bobine engendre une augmentation de sa résistance (R) avec diminution corrélée du courant (i.e. intensité I) dans la bobine (i.e. loi d'Ohm). Une conséquence de cette dérive/diminution du courant (I) est une dérive du degré d'ouverture de l'orifice de l'électrovanne proportionnelle conduisant à une mauvaise régulation de la pression ou du débit du gaz traversant cette électrovanne proportionnelle.

La présente invention s'inscrit dans ce contexte et vise à pallier ce problème afin d'améliorer le contrôle de (ou des) l'électrovanne proportionnelle équipant un appareil de ventilation, i.e. respirateur artificiel ou ventilateurs médical, de manière à fournir une pression ou un débit de gaz plus précis.

Une solution de l'invention concerne un appareil de ventilation ou ventilateur médical comprenant :

- un circuit de gaz interne pour véhiculer un gaz à administrer à un patient,
- au moins une électrovanne proportionnelle comprenant un orifice de passage de gaz à degré d'ouverture ajustable, ladite au moins une électrovanne proportionnelle étant agencée sur ledit circuit de gaz interne et comprenant une bobine alimentée électriquement et
- des moyens de pilotage électroniques configurés pour contrôler le degré d'ouverture de l'orifice de passage de gaz de ladite au moins une électrovanne proportionnelle en agissant sur le courant électrique fourni à la bobine de ladite au moins une électrovanne proportionnelle de manière à délivrer un débit ou une pression de gaz désiré.

De plus, dans l'appareil de ventilation, les moyens de pilotage électroniques comprennent un circuit électronique configuré pour ajuster l'intensité du courant électrique fourni à la bobine en fonction d'une consigne de tension préfixée.

Selon le mode de réalisation considéré, l'appareil de ventilation de l'invention peut comprendre l'une ou plusieurs des caractéristiques suivantes :

- il comprend une source de gaz à administrer à une personne, i.e. un patient.
- la source de gaz comprend une turbine motorisée.
- la turbine motorisée est pilotée par les moyens de pilotage électroniques, en particulier les accélérations et/ou les freinages du moteur.
- alternativement, la source de gaz comprend un apport de gaz via une alimentation pneumatique externe, typiquement une prise murale délivrant du gaz provenant d'une canalisation de gaz, tel une canalisation d'un réseau hospitalier. Dans ce cas, les moyens de pilotage servent aussi à réguler le débit et/ou la pression du gaz fourni par l'alimentation pneumatique externe par action sur des moyens à vannes agencés sur le circuit de gaz, typiquement la branche inspiratoire.
- les moyens de pilotage électroniques comprennent au moins une carte électronique portant au moins un (micro) processeur mettant en œuvre un ou des algorithmes.
- il comprend des moyens d'alimentation en courant électrique.
- les moyens d'alimentation en courant électrique comprennent une batterie rechargeable interne et/ou des moyens de raccordement au secteur (110/220V), tels que câbles électriques, prise électrique ou autres.

- il comprend une coque ou carcasse périphérique.
- les moyens de pilotage électroniques, la ou les électrovannes et le circuit de gaz sont agencés dans la coque de l'appareil.
- le circuit de gaz est à simple branche comprenant uniquement une branche inspiratoire.
- préférentiellement, le circuit de gaz est à double branche, c'est-à-dire qu'il comprend une branche inspiratoire pour amener le gaz respiratoire au patient et une branche expiratoire pour évacuer les gaz expirés riches en $CO_2$.
- il comprend une électrovanne proportionnelle principale agencée sur la branche inspiratoire du circuit de gaz.
- il comprend une électrovanne proportionnelle de PEP (i.e. vanne de PEP) agencée sur la branche expiratoire du circuit de gaz.
- il comprend une électrovanne proportionnelle secondaire agencé sur une ligne d'amenée d'oxygène permettant d'amener de l'oxygène supplémentaire qui peut être ajouté au flux d'air provenant de la source de gaz, typiquement de la turbine, pour l'enrichir en oxygène.
- les moyens de pilotage électroniques sont configurés pour contrôler le degré d'ouverture de l'orifice de passage de gaz de l'électrovanne proportionnelle principale, de l'électrovanne proportionnelle de PEP et/ou de électrovanne proportionnelle secondaire.
- les moyens de pilotage électroniques sont configurés pour contrôler le degré d'ouverture de l'orifice de passage de gaz en régulant le courant dans la bobine de l'électrovanne proportionnelle principale, de l'électrovanne proportionnelle de PEP et/ou de l'électrovanne proportionnelle secondaire.
- une résistance de shunt est agencée dans le circuit électronique, en aval de la bobine, de manière à pouvoir mesurer .
- le circuit électronique comprend une première et une seconde résistances agencées en aval de la bobine et/ou de la résistance de shunt.
- les première et seconde résistances sont configurées pour combiner une tension (Vs) du courant aux bornes de la résistance de shunt à la tension de consigne (Vset) et obtenir une tension combinée (Vfb) alimentant une entrée de contre-réaction du convertisseur DC/DC.
- le circuit de gaz peut aussi comprendre des capteurs de pression et/ou de débit coopérant avec les moyens de pilotage afin de réguler la fourniture de gaz, notamment le pilotage de la source de gaz.
- le circuit de gaz alimente une interface respiratoire patient, tel un masque respiratoire, fournissant le gaz respiratoire aux voies aériennes du patient.
- le circuit de gaz de l'appareil est relié à l'interface respiratoire patient par un ou des tuyaux flexibles.

L'invention va maintenant être mieux comprise grâce à la description détaillée suivante, faite à titre illustratif mais non limitatif, en référence aux figures annexées parmi lesquelles :

Fig. 1 est un schéma de principe d'un mode de réalisation d'un appareil d'assistance respiratoire selon l'invention.
Fig. 2 schématise un mode de réalisation de la boucle de régulation permettant l'asservissement du courant de la bobine de l'électrovanne d'un appareil d'assistance respiratoire selon l'invention, tel celui de Fig. 1.

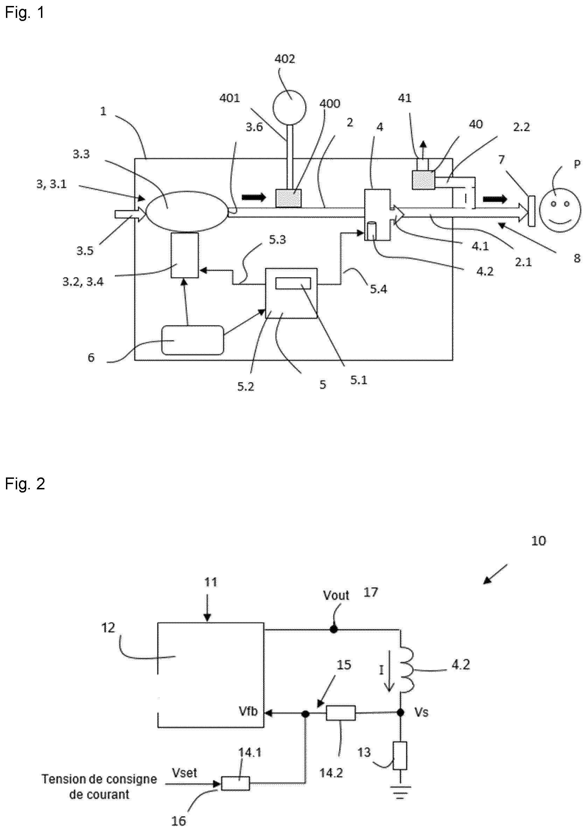

Fig. 1 schématise un mode de réalisation d'appareil d'assistance respiratoire 1 selon l'invention comprenant une coque externe 1.1, dans laquelle sont agencés les composants permettant le bon fonctionnement de l'appareil 1, lorsqu'il est utilisé pour fournir un gaz respiratoire (i.e. gaz à un ou plusieurs constituants), tel de l'air, de l'oxygène, un mélange air/oxygène ou un autre gaz, à un patient.

Ainsi, l'appareil 1 comprend un circuit de gaz interne 2, à savoir un ou des conduits ou passages de gaz, pour véhiculer le gaz à administrer à un patient P au moyen d'une interface respiratoire patient, tel un masque respiratoire 7 ou analogue.

Le circuit de gaz 2 de l'appareil 1 est relié à l'interface respiratoire 7 patient par un ou des tuyaux flexibles 8.

Le circuit de gaz interne 2 est ici à double branche, c'est-à-dire qu'il comprend une branche inspiratoire 2.1 servant à amener le gaz au patient et une branche expiratoire 2.2 servant à récupérer les gaz expirés riches en $CO_2$. Toutefois, un circuit de gaz interne 2 à simple branche est aussi envisageable.

La branche expiratoire 2.2 permet de rejeter les gaz expirés riches en $CO_2$ à l'atmosphère, via un orifice de sortie 41 communiquant fluidiquement avec l'atmosphère ambiante. La branche expiratoire 2.2 comprend aussi une valve de PEP 40 permettant d'ajuster la pression expiratoire (PEP = pression expiratoire positive). La valve de PEP 40 peut être une électrovanne proportionnelle.

Le gaz, ici de l'air, est fourni au circuit de gaz 2, par une source de gaz 3, à savoir ici une turbine 3.1 motorisée (aussi appelée (micro)soufflante, pompe, compresseur ou analogue), c'est-à-dire équipée d'un moteur électrique 3.2 agencé dans un carter de protection 3.4, lequel est surmonté d'une volute 3.3 dans laquelle une roue à ailettes (non visible) est entraînée en rotation par l'axe du moteur 3.2, lors de son fonctionnement. L'air est aspiré par la turbine 3.1 et y pénètre par une entrée d'air 3.5 et en ressort par une sortie 3.6 en communication fluidique avec le circuit de gaz 2.

Toutefois, selon un autre mode de réalisation, la une source de gaz 3 pourrait être une source pneumatique de gaz (non montrée), à savoir une (des) prise murale fournissant le gaz, laquelle serait relié à une canalisation d'amenée de gaz, tel un réseau hospitalier. Dans ce cas, le circuit interne 2 de l'appareil 1 serait équipé de moyens de contrôle à vanne(s) ou valve(s) commandés, comme expliqué ci-après.

Optionnellement, l'appareil 1 peut aussi comprendre une ligne d'amenée d'oxygène 401 permettant d'ajouter de l'oxygène à l'air aspiré par la turbine 3.1. Cette addition d'oxygène peut se faire en amont ou en aval (cf. Fig. 1) de la turbine 3.1 en fonction du mode de réalisation choisi, de préférence en amont de la turbine 3.1 de sorte qu'un mélange air/oxygène pénètre dans la volute 3.3 et soit envoyé ensuite au patient via le circuit de gaz 2. L'oxygène provient d'une source d'oxygène externe 402, telle une bouteille de gaz ou une prise murale fournissant de l'oxygène amené à la prise murale par une canalisation de gaz, tel un réseau hospitalier. Dans ce cas, la ligne d'amenée d'oxygène est généralement pourvue d'une vanne de contrôle, typiquement une électrovanne proportionnelle secondaire 400.

Par ailleurs, comme illustré en Fig. 1, le circuit de gaz interne 2, en particulier la branche inspiratoire 2.1, comprend (au moins) une électrovanne proportionnelle 4 principale comprenant un orifice de passage de gaz 4.1 à degré d'ouverture ajustable, c'est-à-dire à section de passage du gaz variable et modifiable, et une bobine 4.2 alimentée électriquement. L'électrovanne proportionnelle 4 permet de contrôler la pression et/ou le débit du gaz qui la traverse en direction du patient P (sens du gaz schématisé par des flèches noires).

Il est à noter que les autres électrovannes proportionnelles 4, 40, 400, à savoir celle 40 servant au contrôle de la PEP, et celle 400 contrôlant l'apport d'oxygène additionnel peuvent avoir la même configuration et le même fonctionnement que l'électrovanne proportionnelle 4 principale agencée sur la branche inspiratoire 2.1, à savoir un degré d'ouverture ajustable et une bobine alimentée électriquement. Elles sont aussi pilotées par les moyens de pilotage électroniques 5 de l'appareil 1.

En effet, l'appareil 1 comprend aussi des moyens de pilotage électroniques 5 comprenant au moins un microprocesseur 5.1, tel un microcontrôleur, agencé sur une carte électronique 5.2 et mettant en œuvre un ou des algorithmes de pilotage, de contrôle... Les moyens de pilotage 5 permettent notamment de contrôler (5.3) le fonctionnement de la turbine 2, typiquement ses accélérations et décélérations (i.e. freinages) ou les ouvertures des moyens de contrôle à vanne(s) ou valve(s) commandés, dans le cas d'une source pneumatique de gaz, comme évoqué ci-avant.

D'une façon générale, des moyens d'alimentation en courant électrique 6 fournissant le courant électrique nécessaire au bon fonctionnement de l'appareil 1, notamment aux moyens de pilotage électroniques 5. Ils comprennent classiquement une batterie rechargeable interne et/ou des moyens de raccordement au secteur (110/220V), tels que câbles électriques, prise électrique ou autres.

Bien entendu, le circuit de gaz 2 peut aussi comprendre des capteurs de pression et/ou de débit coopérant avec les moyens de pilotage 5 afin de réguler la fourniture de gaz, notamment le pilotage de la source de gaz 3.

Une telle architecture d'appareil d'assistance respiratoire 1 est globalement classique.

Selon l'invention, les moyens de pilotage électroniques 5 permettent aussi de commander ou contrôler le degré d'ouverture de l'orifice de passage de gaz 4.1 de tout ou partie des électrovannes proportionnelles de l'appareil 1, à savoir de l'électrovanne proportionnelle 4 principale agencée sur la branche inspiratoire 2.1, de l'électrovanne proportionnelle de PEP 40 contrôlant la PEP agencée sur la branche expiratoire 2.2 ou encore de l'électrovanne proportionnelle secondaire 400 agencée sur la ligne d'amenée d'oxygène 401 optionnelle.

A titre illustratif, on considère dans le mode de réalisation de Fig. 1, que les moyens de pilotage électroniques 5 contrôlent l'électrovanne proportionnelle 4 principale.

Dans ce cas, le contrôle de l'électrovanne proportionnelle 4 principale se fait, selon l'invention, en agissant sur le courant électrique fourni à la bobine 4.2 de l'électrovanne proportionnelle 4 de manière à délivrer un débit ou une pression de gaz désiré, y compris en cas d'échauffement intempestif de la bobine 4 par effet joule et/ou par augmentation de la température ambiante engendrant une augmentation de la résistance R, comme expliqué ci-après en lien avec Fig. 2.

Pour ce faire, les moyens de pilotage 5 comprennent un circuit électronique 10 configuré pour ajuster l'intensité du courant électrique fourni à la bobine 4.2 en fonction d'une consigne de tension préfixée.

Fig. 2 décrit un mode de réalisation d'un tel circuit électronique 10. Il comprend une entrée d'alimentation électrique 11 en aval de laquelle est agencé un convertisseur de tension 12 Continu /Continu (DC/DC), nommé convertisseur DC/DC. On peut utiliser tout convertisseur DC/DC disponible auprès des fabricants de semi-conducteurs.

L'asservissement du courant I dans la bobine 4.2 de l'électrovanne 4 est réalisée grâce à l'utilisation du convertisseur DC/DC 12 qui est configuré pour permettre une régulation de courant I au lieu d'une régulation de tension.

La tension Vout en sortie du convertisseur DC/DC 12 alimente la bobine 4.2 de l'électrovanne 4 qui est agencée en aval du convertisseur DC/DC 12.

L'intensité du courant I du courant sortant du convertisseur DC/DC 12 et circulant dans la bobine 4.2 est mesurée grâce à une résistance de shunt 13. Dit autrement, la résistance de shunt 13 permet de mesurer le courant I traversant la bobine 4.2 de l'électrovanne 4 principale.

La tension Vs du courant aux bornes de la résistance de shunt 13 est proportionnelle au courant I dans la bobine 4.2.

Le circuit électronique 10 comprend par ailleurs une première 14.1 et une seconde 14.2 résistances, agencées en aval de la résistance de shunt 13.

La tension Vs du courant est combinée à la tension de consigne (Vset), via les deux résistances 14.1,14.2, et on obtient alors une tension combinée, nommée Vfb, qui alimente une entrée de contre-réaction 15 du convertisseur DC/DC 12, permettant au convertisseur DC/DC 12 d'adapter sa tension de sortie Vout afin de satisfaire à la consigne de courant (i.e. intensité souhaitée).

La tension de consigne (Vset) correspond à la tension de commande de l'algorithme de régulation de l'appareil. Elle dépend de la pression ou du débit souhaité qui est fixé par l'utilisateur. Elle est entrée (en 16) au niveau de la première résistance 14.1.

Plus précisément, la détermination de la tension de consigne Vset à partir du courant de consigne I souhaité est opérée comme suit au sein des moyens de pilotage, i.e. microprocesseur.

L'intensité du courant I dans la bobine 4.2 est égale à: $I = Vs / Rs$

où :

- Vs est la tension aux bornes du shunt 13
- Rs est la valeur de résistance de la résistance de shunt 13.

On a alors :

$$Vs = Vfb*(R1+R2)/R1 - Vset*R2/R1$$

où :

- Vfb est la tension de référence de la boucle de contre-réaction interne du convertisseur DC/DC 12
- R1 est la valeur de résistance de la première résistance 14.1
- R2 est la valeur de résistance de la seconde résistance 14.2
- Vset est la valeur de la tension de consigne.

On obtient alors :

$$I = Vfb*(R1+R2)/(R1*Rs) - Vset*(R2/R1*Rs)$$

où : I est le courant souhaité pour réguler le débit ou la pression désiré.

D'où :

$$Vset*(R2/R1*Rs) = Vfb*(R1+R2)/(R1*Rs) - I$$

Donc :

$$Vset = (Vfb*(R1+R2)/(R1*Rs) - I) / (R2/R1*Rs)$$

Autrement dit, le tension Vset est déterminée à partir du courant I souhaité.

La tension Vset est alors appliquée en entrée 16 de la première résistance 14.1, comme expliqué ci-dessus.

A partir de cette tension Vset, les moyens de pilotage peuvent alors contrôler le circuit 10 pour délivrer le courant I souhaité servant à contrôler le degré d'ouverture de l'orifice de passage de gaz 4.1 de ladite au moins une électrovanne proportionnelle 4.

Ceci permet d'améliorer le contrôle de ou des électrovannes proportionnelles 4, 40, 400 équipant l'appareil de ventilation1 et ainsi d'obtenir la fourniture d'une pression ou d'un débit de gaz plus précis par le ventilateur 1.

**Revendications**

1. Appareil de ventilation (1) comprenant :

   - un circuit de gaz interne (2) pour véhiculer un gaz à administrer à un patient,
   - au moins une électrovanne proportionnelle (4, 40, 400) comprenant un orifice de passage de gaz (4.1) à degré d'ouverture ajustable, ladite au moins une électrovanne proportionnelle (4, 40, 400) étant agencée sur ledit circuit de gaz interne (2) et comprenant une bobine (4.2) alimentée électriquement et

- des moyens de pilotage électroniques (5) configurés pour contrôler le degré d'ouverture de l'orifice de passage de gaz (4.1) de ladite au moins une électrovanne proportionnelle (4, 40, 400) en agissant sur le courant électrique fourni à la bobine (4.2) de ladite au moins une électrovanne proportionnelle (4, 40) de manière à délivrer un débit ou une pression de gaz désiré, **caractérisé en ce que** les moyens de pilotage électroniques (5) comprennent un circuit électronique (10) configuré pour ajuster l'intensité du courant électrique fourni à la bobine (4.2) en fonction d'une consigne de tension préfixée (Vset).

2. Appareil selon la revendication 1, **caractérisé en ce que** le circuit électronique (10) comprend un convertisseur de tension (12) Continu /Continu.

3. Appareil selon la revendication 1, **caractérisé en ce qu'**il comprend une électrovanne proportionnelle principale (4) agencée sur une branche inspiratoire (2.1) du circuit de gaz (2).

4. Appareil selon la revendication 1, **caractérisé en ce qu'**il comprend une électrovanne proportionnelle de PEP (40) agencée sur une branche expiratoire (2.2) du circuit de gaz (2).

5. Appareil selon la revendication 1, **caractérisé en ce qu'**il comprend une électrovanne proportionnelle secondaire (400) agencée sur une ligne d'amenée d'oxygène (401).

6. Appareil selon les revendication 1 et 3 à 5, **caractérisé en ce que** les moyens de pilotage électroniques (5) sont configurés pour contrôler le degré d'ouverture de l'orifice de passage de gaz (4.1) de l'électrovanne proportionnelle principale (4), de l'électrovanne proportionnelle de PEP (40) et/ou de l'électrovanne proportionnelle secondaire (400).

7. Appareil selon la revendication 6, **caractérisé en ce que** les moyens de pilotage électroniques (5) sont configurés pour contrôler le degré d'ouverture de l'orifice de passage de gaz (4.1) en régulant le courant dans la bobine (4.2) de l'électrovanne proportionnelle principale (4), de l'électrovanne proportionnelle de PEP (40) et/ou de l'électrovanne proportionnelle secondaire (400).

8. Appareil selon l'une des revendications 1 ou 7, **caractérisé en ce qu'**une résistance de shunt (13) est agencée dans le circuit électronique (10), en aval de la bobine (4.2).

9. Appareil selon la revendication 8, **caractérisé en ce que** le circuit électronique (10) comprend une première et une seconde résistances (14.1,14.2) agencées en aval de la bobine (4.2) et/ou la résistance de shunt (13).

10. Appareil selon la revendication 9, **caractérisé en ce que** les première et seconde résistances (14.1,14.2) sont configurées pour combiner une tension (Vs) du courant aux bornes de la résistance de shunt (13) à la tension de consigne (Vset) et obtenir une tension combinée (Vfb) alimentant une entrée de contre-réaction (15) du convertisseur DC/DC (12).

**Revendications modifiées conformément à la règle 137(2) CBE.**

1. Appareil de ventilation (1) comprenant :

   - un circuit de gaz interne (2) pour véhiculer un gaz à administrer à un patient,
   - au moins une électrovanne proportionnelle (4, 40, 400) comprenant un orifice de passage de gaz (4.1) à degré d'ouverture ajustable, ladite au moins une électrovanne proportionnelle (4, 40, 400) étant agencée sur ledit circuit de gaz interne (2) et comprenant une bobine (4.2) alimentée électriquement,
   - qu'une résistance de shunt (13) est agencée dans le circuit électronique (10), en aval de la bobine (4.2),
   - une première et une seconde résistances (14.1,14.2) agencées en aval de la bobine (4.2) et/ou la résistance de shunt (13),
   et
   - des moyens de pilotage électroniques (5) configurés pour contrôler le degré d'ouverture de l'orifice de passage de gaz (4.1) de ladite au moins une électrovanne proportionnelle (4, 40, 400) en agissant sur le courant électrique fourni à la bobine (4.2) de ladite au moins une électrovanne proportionnelle (4, 40) de manière à délivrer un débit ou une pression de gaz désiré, **caractérisé en ce que** les moyens de pilotage électroniques (5) comprennent un circuit électronique (10) configuré pour ajuster l'intensité du courant électrique fourni à la bobine (4.2) en fonction d'une consigne de tension préfixée (Vset) déterminée en fonction de la résistance de shunt (13), des premières et secondes résistances (14.1, 14.2) et de l'intensité du courant électrique à fournir.

2. Appareil selon la revendication 1, **caractérisé en ce que** le circuit électronique (10) comprend un convertisseur de tension (12) Continu /Continu.

3. Appareil selon la revendication 1, **caractérisé en ce qu'**il comprend une électrovanne proportionnelle principale (4) agencée sur une branche inspiratoire (2.1) du circuit de gaz (2).

4. Appareil selon la revendication 1, **caractérisé en ce qu'**il comprend une électrovanne proportionnelle de PEP (40) agencée sur une branche expiratoire (2.2) du circuit de gaz (2).

5. Appareil selon la revendication 1, **caractérisé en ce qu'**il comprend une électrovanne proportionnelle secondaire (400) agencée sur une ligne d'amenée d'oxygène (401).

6. Appareil selon les revendication 1 et 3 à 5, **caractérisé en ce que** les moyens de pilotage électroniques (5) sont configurés pour contrôler le degré d'ouverture de l'orifice de passage de gaz (4.1) de l'électrovanne proportionnelle principale (4), de l'électrovanne proportionnelle de PEP (40) et/ou de l'électrovanne proportionnelle secondaire (400).

7. Appareil selon la revendication 6, **caractérisé en ce que** les moyens de pilotage électroniques (5) sont configurés pour contrôler le degré d'ouverture de l'orifice de passage de gaz (4.1) en régulant le courant dans la bobine (4.2) de l'électrovanne proportionnelle principale (4), de l'électrovanne proportionnelle de PEP (40) et/ou de l'électrovanne proportionnelle secondaire (400).

8. Appareil selon la revendication 2, **caractérisé en ce que** les première et seconde résistances (14.1,14.2) sont configurées pour combiner une tension (Vs) du courant aux bornes de la résistance de shunt (13) à la tension de consigne (Vset) et obtenir une tension combinée (Vfb) alimentant une entrée de contre-réaction (15) du convertisseur DC/DC (12).

Fig. 1

Fig. 2

Europäisches
Patentamt

European
Patent Office

Office européen
des brevets

**RAPPORT DE RECHERCHE EUROPEENNE**

Numéro de la demande

EP 25 18 2596

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | WO 00/32261 A1 (BUNNEL INC [US]) 8 juin 2000 (2000-06-08) * le document en entier, et en particulier page 11 lignes 6 à 19, et les figures 2 et 3 * ----- | 1-10 | INV. A61M16/00 A61M16/20 |
| X | WO 2014/210552 A1 (CAREFUSION 303 INC [US]) 31 décembre 2014 (2014-12-31) * le document en entier, et en particulier les paragraphes [0040]-[0047] et les figures 1, 4A-4B * ----- | 1-10 | |
| X | WO 2014/210566 A2 (CAREFUSION 303 INC [US]) 31 décembre 2014 (2014-12-31) * le document en entier, et en particulier les figures 10, 13A-13B * ----- | 1-10 | |
| A | US 5 265 594 A (OLSSON SVEN-GUNNAR [SE] ET AL) 30 novembre 1993 (1993-11-30) * le document en entier * ----- | 1-10 | |
| A | US 6 536 433 B1 (CEWERS GOERAN [SE]) 25 mars 2003 (2003-03-25) * le document en entier * ----- | 1-10 | **DOMAINES TECHNIQUES RECHERCHES (IPC)** A61M |
| A | US 2021/299375 A1 (JENARO JULIO [IE]) 30 septembre 2021 (2021-09-30) * le document en entier * ----- | 1-10 | |

Le présent rapport a été établi pour toutes les revendications

| Lieu de la recherche | Date d'achèvement de la recherche | Examinateur |
|---|---|---|
| Munich | 16 juillet 2025 | Azaïzia, Mourad |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 18 2596

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-07-2025

| Document brevet cité au rapport de recherche | | | Date de publication | Membre(s) de la famille de brevet(s) | | Date de publication |
|---|---|---|---|---|---|---|
| WO | 0032261 | A1 | 08-06-2000 | US | 6279574 B1 | 28-08-2001 |
| | | | | WO | 0032261 A1 | 08-06-2000 |
| WO | 2014210552 | A1 | 31-12-2014 | AU | 2014302094 A1 | 24-12-2015 |
| | | | | BR | 112015031635 A2 | 25-07-2017 |
| | | | | CA | 2914858 A1 | 31-12-2014 |
| | | | | CN | 105451797 A | 30-03-2016 |
| | | | | CN | 107252514 A | 17-10-2017 |
| | | | | EP | 3013398 A1 | 04-05-2016 |
| | | | | ES | 2636654 T3 | 06-10-2017 |
| | | | | JP | 2016523662 A | 12-08-2016 |
| | | | | MX | 349592 B | 04-08-2017 |
| | | | | RU | 2015155882 A | 30-06-2017 |
| | | | | US | 2015000662 A1 | 01-01-2015 |
| | | | | US | 2016346499 A1 | 01-12-2016 |
| | | | | WO | 2014210552 A1 | 31-12-2014 |
| WO | 2014210566 | A2 | 31-12-2014 | AU | 2014302108 A1 | 21-01-2016 |
| | | | | BR | 112015032320 A2 | 25-07-2017 |
| | | | | CA | 2915686 A1 | 31-12-2014 |
| | | | | CN | 105358202 A | 24-02-2016 |
| | | | | CN | 107362424 A | 21-11-2017 |
| | | | | EP | 3013400 A2 | 04-05-2016 |
| | | | | EP | 3219350 A1 | 20-09-2017 |
| | | | | EP | 3689404 A1 | 05-08-2020 |
| | | | | ES | 2632484 T3 | 13-09-2017 |
| | | | | JP | 2016526639 A | 05-09-2016 |
| | | | | RU | 2015155886 A | 30-06-2017 |
| | | | | WO | 2014210566 A2 | 31-12-2014 |
| US | 5265594 | A | 30-11-1993 | EP | 0483401 A1 | 06-05-1992 |
| | | | | ES | 2062258 T3 | 16-12-1994 |
| | | | | JP | H0653175 B2 | 20-07-1994 |
| | | | | JP | H04263867 A | 18-09-1992 |
| | | | | US | 5265594 A | 30-11-1993 |
| US | 6536433 | B1 | 25-03-2003 | EP | 1048313 A2 | 02-11-2000 |
| | | | | JP | 2000354632 A | 26-12-2000 |
| | | | | US | 6536433 B1 | 25-03-2003 |
| US | 2021299375 | A1 | 30-09-2021 | AUCUN | | |

Pour tout renseignement concernant cette annexe : voir Journal Officiel de l'Office européen des brevets, No.12/82

EPO FORM P0460

# EP 4 681 755 A1

**RÉFÉRENCES CITÉES DANS LA DESCRIPTION**

**Documents brevets cités dans la description**

- WO 0032261 A
- WO 2024210552 A
- WO 2024210566 A